# EUROPEAN PATENT SPECIFICATION

(11) **EP 3 875 140 B1**
(45) Date of publication and mention of the grant of the patent: **31.07.2024**
(21) Application number: 19877717.9
(22) Date of filing: 30.10.2019
(51) Int. Cl.: A61M 37/00

(54) **MICRONEEDLE ARRAY UNIT**
MIKRONADELANORDNUNGSEINHEIT
UNITÉ RANGÉE DE MICRO-AIGUILLES

(30) Priority: 31.10.2018 JP 2018204830
(43) Date of publication of application: 08.09.2021
(73) Proprietor: FUJIFILM Corporation, Tokyo 106-8620 (JP)
(72) Inventor: YOSHIDA, Junya, Ashigarakami-gun, Kanagawa 258-8577 (JP); KABATA, Koki, Ashigarakami-gun, Kanagawa 258-8577 (JP); KOBAYASHI, Yuka, Ashigarakami-gun, Kanagawa 258-8577 (JP)
(74) Representative: Klunker IP Patentanwälte PartG mbB
(86) International application number: PCT/JP2019/042462
(87) International publication number: WO 2020/090846

(56) References cited:
- WO-A1-2012/075375
- WO-A2-2009/107806
- JP-A- 2016 131 619
- JP-A- 2018 191 783
- JP-B2- 5 553 612
- US-A1- 2009 198 189
- US-A1- 2009 198 189
- US-A1- 2010 274 203
- US-A1- 2012 029 434
- US-A1- 2016 325 081
- US-A1- 2016 325 082
- US-A1- 2016 354 589

## Description

### Field of the Invention

The present invention relates to a micro-needle array unit.

### Description of the Related Art

In recent years, a micro-needle array has been known as a new dosage form which enables administration of a drug into the skin without pain. The micro-needle array is formed such that biodegradable micro-needles (also referred to as fine needles or microneedles) containing a drug are arranged in an array. By pressing this micro-needle array against the skin, the skin is punctured by each micro-needle. The micro-needles which have punctured the skin are absorbed in the skin, and the drug contained in each micro-needle is administered into the skin.

A container (also referred to as an applicator) that is pressed against the skin in a state of accommodating a micro-needle array in order to protect micro-needles until the skin is punctured by the micro-needles and enables the micro-needles to easily puncture the skin (Patent Document 1). Specifically, Patent Document 1 discloses, in paragraph 0041, in a case where the applicator that holds the micro-needle array is disposed on the skin, the pressure is applied to the holder, for example, by applying the pressure thereto using a finger, the applicator is inverted so that the holder comes into contact with the skin, and thus the micro-needles penetrate into the skin.
Patent Document 1 discloses a micro-needle array unit according to the preamble of claim 1. The puncture instrument of this prior art unit has a piston having a relatively large area and a shaft having a small diameter. A prestressed spring may be released to make the shaft and the piston move against the micro-needle array so as to press it into contact with a skin. Said spring is accommodated in a handle which carries the micro-needle array unit.

### Prior Art Documents

### Patent Documents

Patent Document 1: JP5553612B
Patent Document 2: WO 2012/075375

### SUMMARY OF THE INVENTION

The container of Patent Document 1 elastically deforms an outer portion that is integrated with an inner portion that holds the micro-needle array. Therefore, the size of the container is increased in some cases. Further, in a case where the pressure is applied to the applicator holding the micro-needle array using a finger, the pressure required for the micro-needles to penetrate into the skin may not be obtained depending on the shape or softness of the finger.

The present invention has been made in consideration of the above-described circumstances, and an object thereof is to provide a micro-needle array unit that can be miniaturized and allow a micro-needle array to sufficiently puncture the skin even in a case of being pressed by a finger.

A micro-needle array unit according to a first aspect, comprising: a micro-needle array which has a sheet and a plurality of needles arranged inside an outer peripheral surface of one surface of the sheet; a container which accommodates the micro-needle array and includes an accommodating portion having an opening and a protrusion that supports the outer peripheral surface of the micro-needle array, a deformable portion disposed on a side opposite to the opening and integrated with the accommodating portion, and a flange portion that is integrated with the accommodating portion and brought into contact with a skin; a lid which seals the opening of the container; and a three-dimensional puncture instrument which has two vertically different areas, in which in a case where an external force applied in a direction of the opening by a smaller area between two vertically different areas of the puncture instrument is received, the deformable portion is deformed, and the other surface of the micro-needle array is pressed, the micro-needle array passes through the protrusion and is pushed out of the accommodating portion due to the pressing of the other surface, and the deformable portion presses the micro-needle array while maintaining a deformed state thereof.

In the micro-needle array unit according to a second aspect, the protrusion is disposed closer to a side of the opening than a side of the deformable portion.

In the micro-needle array unit according to a third aspect, the deformable portion has a convex shape with a vertex portion separated from the micro-needle array.

In the micro-needle array unit according to a fourth aspect, the convex shape is a dome shape or a cone shape.

In the micro-needle array unit according to a fifth aspect, a plurality of the protrusions are arranged at an equal interval in the accommodating portion.

In the micro-needle array unit according to a sixth aspect, the protrusion is formed as a continuous protrusion disposed in the accommodating portion.

In the micro-needle array unit according to a seventh aspect, the flange portion has an adhesive on a side to be brought into contact with the skin.

The micro-needle array unit according to an eighth aspect further comprises a flat plate on a side of the other surface of the micro-needle array.

In the micro-needle array unit according to a ninth aspect, the flange portion is provided in an entire circumference of the accommodating portion.

The micro-needle array unit according to a tenth aspect, the flange portion includes a bent portion that is bent to a side of the deformable portion.

In the micro-needle array unit according to an eleventh aspect, the bent flange portion is disposed at a position beyond the deformable portion with reference to the opening of the accommodating portion.

In the micro-needle array unit according to a twelfth aspect, in the three-dimensional puncture instrument having two vertically different areas, a diameter of a surface of one of the two vertically different areas is in a range of 3 mm to 10 mm, and a diameter of a surface of the other of the two vertically different areas is in a range of 15 mm to 30 mm.

According to the present invention, it is possible to provide a micro-needle array unit that can be miniaturized and allow a micro-needle array to sufficiently puncture the skin even in a case of being pressed by a finger.

### BRIEF DESCRIPTION OF THE DRAWINGS

Fig. 1 is a perspective view illustrating a micro-needle array unit.
Fig. 2 is a cross-sectional view taken along the line I-I of the micro-needle array unit of Fig. 1.
Fig. 3 is a perspective view illustrating a micro-needle array.
Fig. 4 is a bottom view illustrating the micro-needle array unit of Fig. 1.
Fig. 5 is a bottom view illustrating another micro-needle array unit.
Fig. 6 is a perspective view of the micro-needle array unit illustrating a step of puncturing the skin with the micro-needle array.
Fig. 7 is a perspective view of the micro-needle array unit illustrating the step of puncturing the skin with the micro-needle array.
Fig. 8 is a cross-sectional view of the micro-needle array unit illustrating the step of puncturing the skin with the micro-needle array.
Fig. 9 is a cross-sectional view of the micro-needle array unit illustrating the step of puncturing the skin with the micro-needle array.
Fig. 10 is a cross-sectional view of the micro-needle array unit illustrating the step of puncturing the skin with the micro-needle array.
Fig. 11 is a cross-sectional view illustrating a micro-needle array unit in another form.
Fig. 12 is a bottom view illustrating a micro-needle array unit in still another form.
Fig. 13 is a bottom view illustrating a micro-needle array unit in still another form.
Fig. 14 is a cross-sectional view illustrating a micro-needle array unit in still another form.
Fig. 15 is a cross-sectional view illustrating a micro-needle array unit in still another form.
Figs. 16A to 16D are perspective views and top views illustrating puncture instruments.
Figs. 17A and 17B are perspective views and top views illustrating puncture instruments in other forms.

### DESCRIPTION OF THE PREFERRED EMBODIMENTS

Hereinafter, preferred embodiments of the present invention will be described with reference to the accompanying drawings. The present invention will be described based on the following preferred embodiments. Modifications can be made according to various techniques without departing from the scope of the present invention and embodiments other than the following embodiments can be employed. Therefore, all modifications within the scope of the present invention are included in the scope of the appended claims. In the present invention, the terms describing geometric conditions or shapes (for example, "upper", "lower", "upper end", "lower end", "flat plate", and "frustum") are not used in a strict sense, but used to show relative positional relationships and the ranges where movements or functions can be expected.

A micro-needle array unit according to an embodiment is a micro-needle array unit comprising a micro-needle array, a container that allows protrusions to support the micro-needle array, and a lid that seals the opening of the container, in which an external force is applied to the container from a side opposite to the opening so that the container is partially deformed, the micro-needle array is pushed out of the container, and the micro-needle array is pressed by the deformed container. Hereinafter, preferred embodiments will be described.

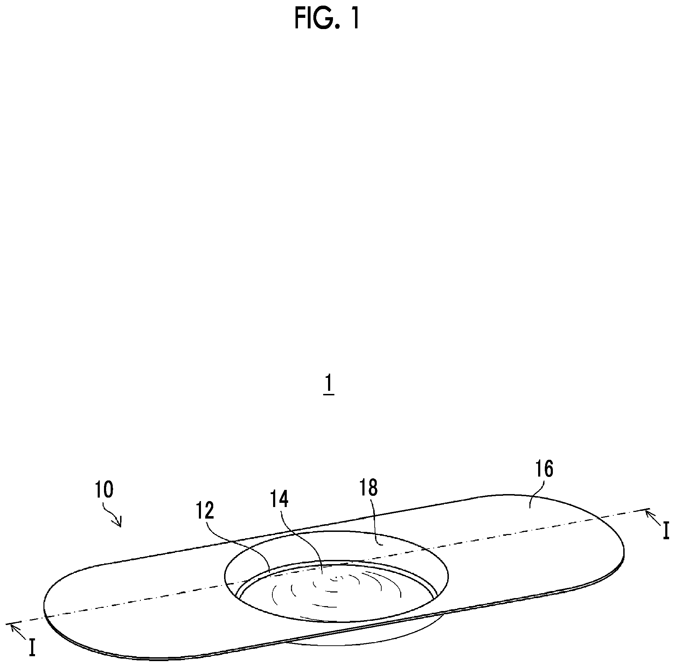

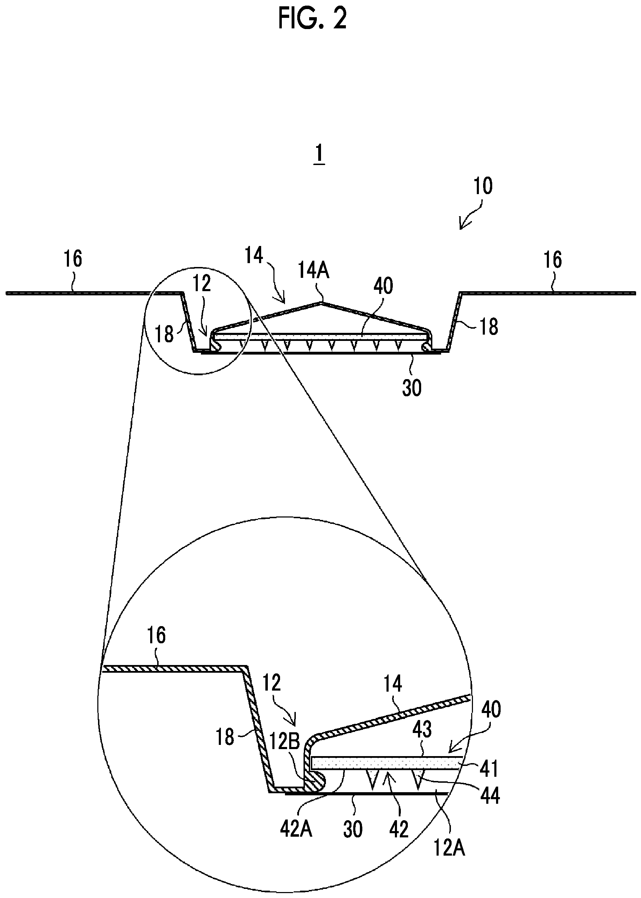

Fig. 1 is a perspective view illustrating a micro-needle array unit, and Fig. 2 is a cross-sectional view taken along the line I-I in Fig. 1. A micro-needle array unit 1 will be described with reference to Figs. 1 and 2.

As illustrated in Fig. 1, the micro-needle array unit 1 comprises a container 10. The container 10 comprises an accommodating portion 12 for accommodating a micro-needle array, a deformable portion 14 integrated with the accommodating portion 12, and a flange portion 16 which is integrated with the accommodating portion 12 and bent by a bent portion 18.

The accommodating portion 12 and the deformable portion 14 of the container 10 respectively have a circular shape in a plan view. The flange portion 16 of the container 10 has a racetrack shape (shape formed by combining two semicircles and two straight lines) in a plan view. However, the shapes of the accommodating portion 12, the deformable portion 14, and the flange portion 16 are not limited. In the embodiment, the flange portion 16 is provided in the entire circumference of the accommodating portion 12. The entire circumference means that the entire circumference of the accommodating portion 12 is enclosed by the flange portion 16. The flange portion 16 is not necessarily provided in the entire circumference of the accommodating portion 12. Further, it is preferable that the flange portion 16 contains an adhesive on the surface to be brought into contact with the skin. The container 10 is attached to the skin because of the adhesive of the flange portion 16. Even in a case where the flange portion 16 does not contain an adhesive, the container 10 is attached to the skin because of an adhesive applied to the skin. Further, the container 10 is attached to the skin by attaching another member (medical tape) or the like from above the container 10.

As illustrated in Fig. 2, the accommodating portion 12 has an internal space defined by an inner wall and an opening 12A. The opening 12A of the accommodating portion 12 is sealed by a lid 30. The accommodating portion 12 comprises protrusions 12B that are arranged on the inner wall and protrude to the internal space. The accommodating portion 12 has a cylindrical shape according to the embodiment, but the shape of the accommodating portion 12 is not limited as long as a micro-needle array 40 can be accommodated therein.

The deformable portion 14 is disposed on a side opposite to the opening 12A and integrated with the accommodating portion 12. In the embodiment, for example, the deformable portion 14 is formed in a convex shape with a vertex portion 14A separated from the micro-needle array 40. The vertex portion 14A of the deformable portion 14 indicates a portion furthest from the micro-needle array 40 in the deformable portion 14, and the convex shape indicates that the vertex portion 14A is not positioned in the internal space of the accommodating portion 12. The deformable portion 14 may have a plurality of vertex portions 14A. The integration indicates a state where the accommodating portion 12 and the deformable portion 14 are connected with each other. For example, in a case where the accommodating portion 12 is integrated with the deformable portion 14, this integration can be realized by separately molding the accommodating portion 12 and the deformable portion 14, fitting the accommodating portion 12 and the deformable portion 14 to each other, and welding the accommodating portion 12 and the deformable portion 14. In a case where the accommodating portion 12 is integrated with the deformable portion 14, the integration may be carried out before or after the accommodation of the micro-needle array 40 in the accommodating portion 12. In the case where the accommodating portion 12 is integrated with the deformable portion 14, the integration can be realized by integrally molding the accommodating portion 12 and the deformable portion 14. However, the integration method is not limited to these methods.

The deformable portion 14 can be formed in a cone shape.

According to the embodiment, the deformable portion 14 has a conical shape. Further, the deformable portion 14 may have, for example, an internal space, and the internal space of the deformable portion 14 can communicate with the internal space of the accommodating portion 12. The accommodating portion 12 has a structure in which the side opposite to the opening 12A is closed by the deformable portion 14. The type of the cone shape includes a conical shape, a pyramid shape, and a frustum shape.

The flange portion 16 is integrated with the accommodating portion 12 and brought into contact with the skin as described below. According to the embodiment, the flange portion 16 extends to the outside from the position of the opening 12A of the accommodating portion 12 and is bent to the side of the deformable portion 14 by the bent portion 18. According to the embodiment, the flange portion 16 is disposed at a position beyond the vertex portion 14A of the deformable portion 14 with respect to the opening 12A of the accommodating portion 12. The flange portion 16 is formed to be parallel to the sheet of the micro-needle array 40. The concept of parallel includes parallel and substantially parallel. As described below, the shape of the flange portion 16 is not particularly limited as long as the flange portion comes into contact with the skin. In a case where the accommodating portion 12 is integrated with the flange portion 16, the same method used for integration of the accommodating portion 12 with the deformable portion 14 can be applied.

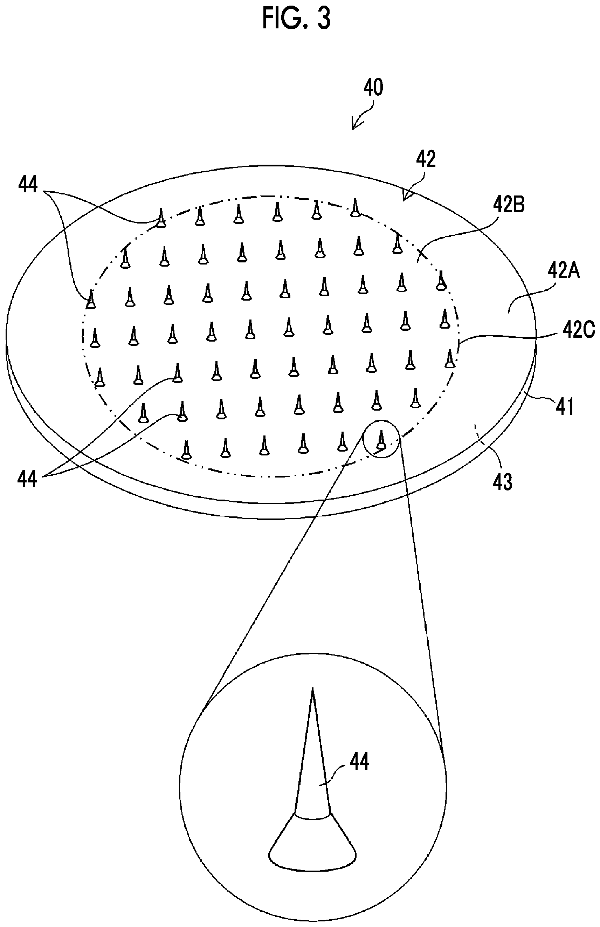

A typical structure of the micro-needle array 40 will be described with reference to Fig. 3. Fig. 3 is a perspective view illustrating the micro-needle array 40. As illustrated in Fig. 3, the micro-needle array 40 comprises a circular sheet 41 having one surface 42 and the other surface 43 which oppose each other and a plurality of needles 44 arranged on the one surface 42 of the sheet 41. The needles 44 constitute micro-needles. The plurality of needles 44 are arranged in a micro-needle region 42B inside an outer peripheral surface 42A of the one surface 42. As illustrated in Fig. 3, the boundary between the outer peripheral surface 42A and the micro-needle region 42B is an imaginary line 42C that connects the needles 44, which are arranged on the outermost side of the micro-needle region 42B, from among the plurality of needles 44. According to the embodiment, an example in which the sheet 41 has a circular shape has been described, but the sheet 41 may have a rectangular shape.

The shape and the size of the sheet 41 and the needles 44 may be selected according to the applications of the micro-needle array 40. Further, the sheet 41 and the needles 44 may be formed of the same material or different materials. The micro-needle array 40 can be produced by integrally molding the sheet 41 and the needles 44, but the sheet 41 and the needles 44 may be molded separately.

The needles 44 respectively have, for example, a substantially cone shape, but may have a columnar shape or a frustum shape. According to the embodiment, the needles 44 are formed in order of a truncated cone portion and a cone from the one surface 42 toward the tip, but the shape thereof is not particularly limited as long as the skin can be punctured by the needles. It is preferable that the needles 44 are arranged in an array in a state of columns (lateral rows) and rows (vertical rows) at equal intervals.

Each needle 44 may be formed of a metal material, but it is preferable that each needle 44 is formed of a material that is dissolved after the skin or the mucous membrane is punctured by the needles so that the needles are inserted into the body. Accordingly, as the material constituting the needles 44, a water-soluble polymer is preferable and polysaccharides are more preferable. As the material constituting the needles 44, it is preferable that the needles are formed of at least one material selected from the group consisting of hydroxyethyl starch, dextran, chondroitin sulfate, sodium hyaluronate, carboxymethyl cellulose, polyvinylpyrrolidone, polyoxyethylene polyoxypropylene glycol, and polyethylene glycol.

Each needle 44 is coated with or contains a drug. Each needle 44 penetrates the skin to puncture the body in a case of attaching the sheet 41 to the surface of the skin. In a case where each needle 44 is coated with the drug, the drug is administered into the body from the surface of each needle 44. Further, in a case where the drug is contained in each needle 44, since each needle 44 is formed of a material that is dissolved after each needle 44 is used to puncture the body, the drug in the needle 44 is administered into the body due to the dissolution of the needle 44 in the body.

The sheet 41 of the micro-needle array 40 has a diameter of 10 mm to 30 mm and a thickness of 0.1 mm to 5 mm. Further, each needle 44 has a length of 0.2 mm to 1.5 mm. Further, the number of needles 44 to be arranged on the one surface 42 of the sheet 41 is in a range of 4 to 1000. However, the values are not limited thereto.

As illustrated in Fig. 2, the protrusion 12B supports the outer peripheral surface 42A of the micro-needle array 40 in a state where each tip of the needle 44 is directed to the gravity direction. The micro-needle array 40 is accommodated in the internal space of the accommodating portion 12 by the protrusion 12B in a state where the needles 44 face the opening 12A.

The other surface 43 of the micro-needle array 40 opposes the deformable portion 14. According to the embodiment, the deformable portion 14 has a conical shape and the inner diameter of the deformable portion 14 decreases toward the vertex portion 14A. Even in a case where the container 10 is vibrated during the transport or the like, movement of the micro-needle array 40 is restricted by the protrusions 12B and the deformable portion 14. The micro-needle array unit 1 according to the embodiment is not provided with an adhesive for fixing the micro-needle array 40, but the micro-needle array 40 may be fixed by disposing an adhesive inside the accommodating portion 12.

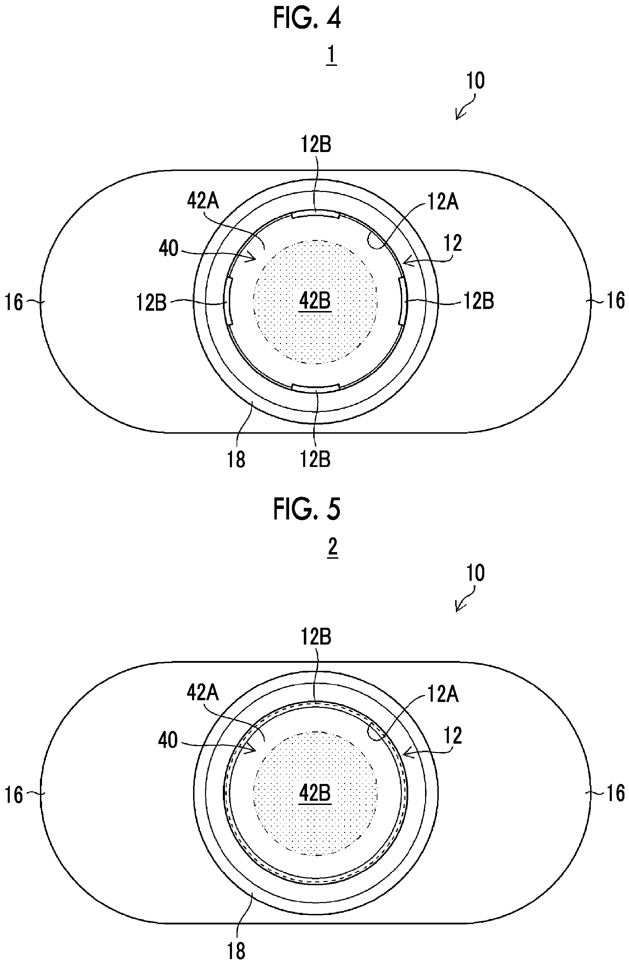

Fig. 4 is a bottom view illustrating the micro-needle array unit 1. In the micro-needle array unit 1, the lid 30 is not illustrated for the sake of understanding. Fig. 4 illustrates a state where the micro-needle array 40 is exposed from the opening 12A. As illustrated in Fig. 4, four protrusions 12B are provided on the inner wall of the accommodating portion 12 at equal intervals. Four protrusions 12B support the outer peripheral surface 42A of the micro-needle array 40.

Fig. 5 is a bottom view illustrating a micro-needle array unit 2 in another form. In the micro-needle array unit 1, the lid 30 is not illustrated for the sake of understanding. As illustrated in Fig. 5, the protrusion 12B is continuously provided along the inner wall of the accommodating portion 12. One continuous protrusion 12B supports the outer peripheral surface 42A of the micro-needle array 40.

The position where the protrusions are arranged and the number of the protrusions 12B are not limited as long as the outer peripheral surface 42A of the micro-needle array 40 can be supported in a state where the tips of the needles 44 are directed to the gravity direction.

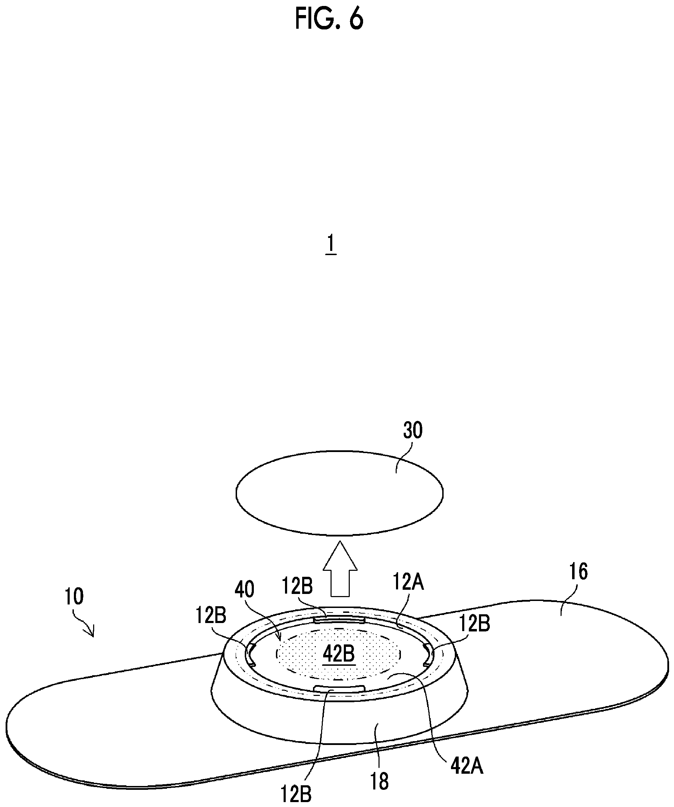

Next, a step of puncturing the skin with the micro-needle array 40 using the micro-needle array unit 1 will be described with reference to Figs. 6 to 10. The configurations which are the same as the configurations described in Figs. 1 to 5 are denoted by the same reference numerals, and the description thereof will not be provided.

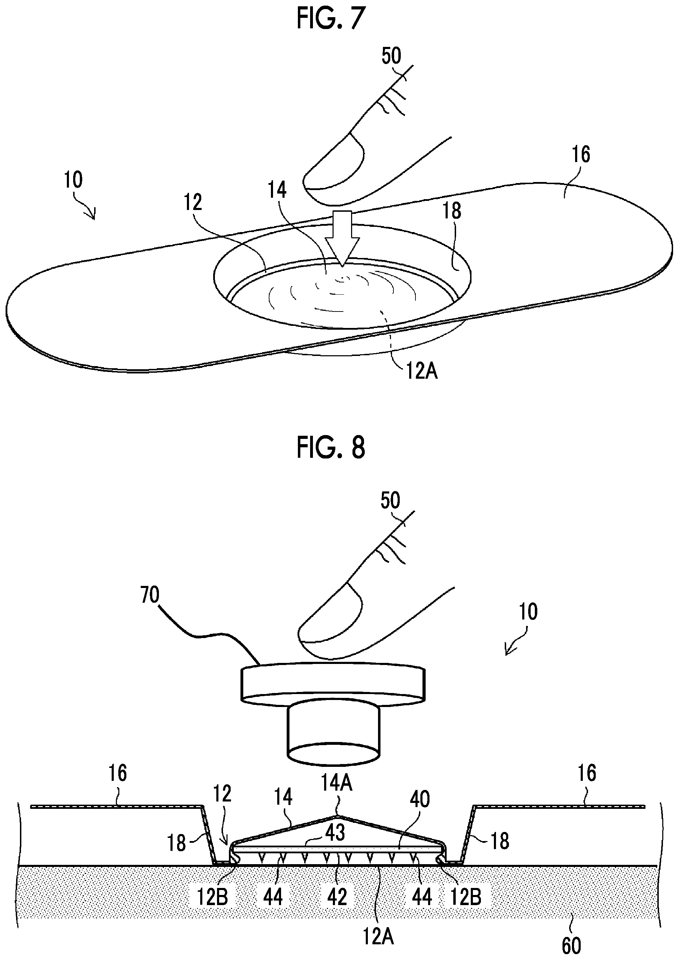

Figs. 6 and 7 are perspective views of the micro-needle array unit illustrating a step of puncturing the skin with the micro-needle array 40. Figs. 8 to 10 are cross-sectional views of the micro-needle array unit 1 illustrating the step of puncturing the skin with the micro-needle array 40.

As illustrated in Fig. 6, the lid 30 that seals the opening 12A of the accommodating portion 12 is peeled off from the container 10. The micro-needle region 42B of the micro-needle array 40 is exposed from the opening 12A. Until the micro-needle array unit 1 is used, the lid 30 protects the needles 44 (not illustrated) of the micro-needle region 42B from damage. It is preferable that the lid 30 has a knob in order to facilitate the peeling.

As illustrated in Fig. 7, the container 10 is positioned on the skin. The opening 12A of the accommodating portion 12 is positioned toward the skin so that the needles 44 (not illustrated) of the micro-needle array 40 are directed to the skin. An external force in a direction (direction indicated by an arrow in the figure) of the opening 12A is applied to the deformable portion 14 by a finger 50.

Fig. 8 is a cross-sectional view of Fig. 7. As illustrated in Fig. 8, the container 10 is positioned on the skin 60. A portion of the flange portion 16 protruding to the outside from the accommodating portion 12 is brought into contact with the skin 60. In order to apply an external force in the direction of the opening 12A to the deformable portion 14, the finger 50 is positioned at a position separated from the vertex portion 14A of the deformable portion 14 through a three-dimensional puncture instrument 70 having two vertically different areas (hereinafter, also referred to as the puncture instrument 70). The micro-needle array 40 is supported by the protrusions 12B and positioned in the internal space of the accommodating portion 12.

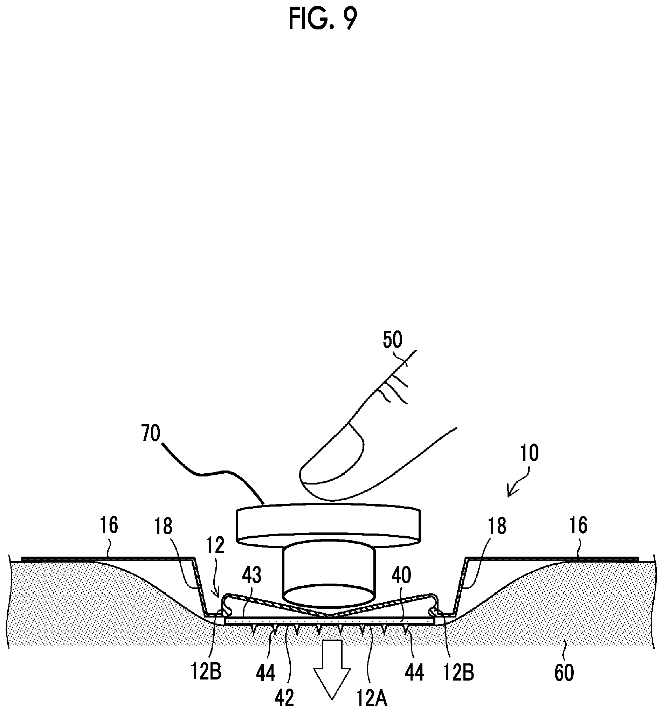

As illustrated in Fig. 9, the deformable portion 14 is pressed against the skin 60 by pressing the puncture instrument 70 with the finger 50. The deformable portion 14 is deformed by receiving the external force in the direction of the opening 12A. The deformable portion 14 presses the other surface 43 of the micro-needle array 40. By pressing the other surface 43, the micro-needle array 40 passes through the protrusions 12B and is pushed out of the accommodating portion 12. The micro-needle array 40 passes through the opening 12A, and the needles 44 of the micro-needle array 40 puncture the skin 60. It is preferable that the protrusions 12B are elastically deformed in a case of the passage of the micro-needle array 40. The elastically deformable protrusions 12B enable the micro-needle array 40 to be easily inserted into the accommodating portion 12 and to be easily pushed out of the accommodating portion 12.

Along with the application of the external force to the deformable portion 14, the skin 60 is moved until the skin comes into contact with the flange portion 16. In a case where the surface of the flange portion 16 which opposes the skin 60 is provided with an adhesive, the flange portion 16 is attached to the skin 60.

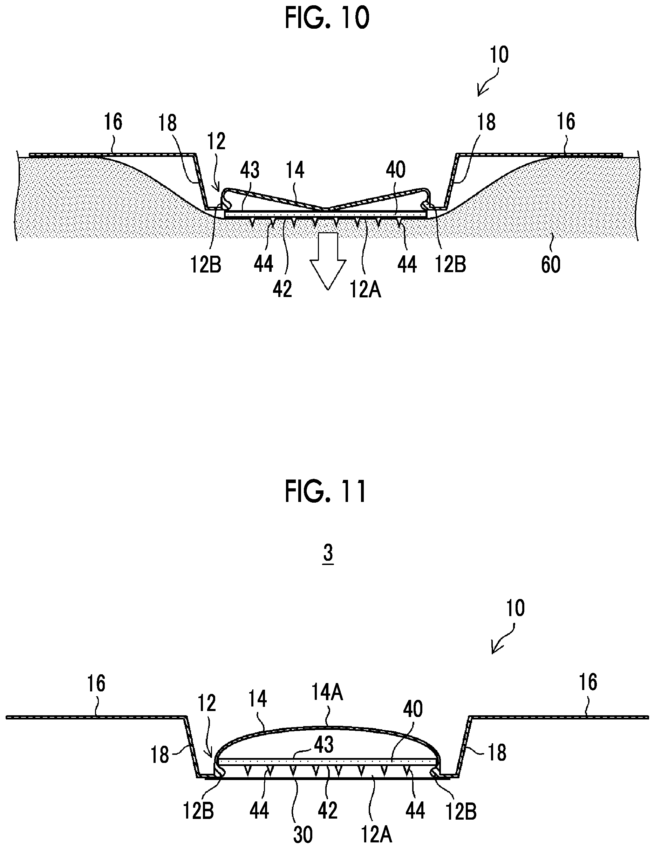

As illustrated in Fig. 10, the deformable portion 14 is deformed by the external force. Even after the external force is removed, the deformable portion 14 maintains the deformed shape. The deformed deformable portion 14 presses the micro-needle array 40 toward the skin 60.

After the puncture, since the micro-needle array 40 is pressed by the deformable portion 14 of the container 10 until the drug of the micro-needle array 40 is administered, falling of the micro-needle array 40 off the skin 60 without pressing of the finger 50 is prevented.

According to the embodiment, since the flange portion 16 includes the bent portion 18, a step is formed between the puncture position of the micro-needle array 40 and the flange portion 16. Because of the step of the bent portion 18, the micro-needle array 40 is pushed down further than the skin 60 that comes into contact with the flange portion 16. By pushing the micro-needle array 40 down, a force of the skin 60 to return is increased so that a mutual pressing force between the skin 60 and the micro-needle array 40 is increased. Further, the needles 44 of the micro-needle array 40 enter a state of easily puncturing the skin 60. It is preferable that the deformed deformable portion 14 is not deformed even in a case of receiving a pressure from the skin 60. The deformable portion 14 is capable of continuously pressing the micro-needle array 40.

According to the embodiment, the deformable portion 14 of the container 10 is disposed inside the projection surface of the accommodating portion 12, which accommodates the micro-needle array 40, in the central axis direction. Therefore, the disposition of the accommodating portion 12 and the deformable portion 14 in the container 10 leads to a decrease in size of the container 10. As the result, the size of the micro-needle array unit 1 is decreased (see Fig. 2). Consequently, the skin 60 is easily punctured by the micro-needle array 40.

It is preferable that the container 10 and the lid 30 that constitute the micro-needle array unit 1 illustrated in Fig. 2 are formed of, for example, a polyethylene resin, a polypropylene resin, or a mixture thereof. However, the materials are not limited thereto. It is preferable that these materials respectively satisfy the "Specification of Plastic Container for Aqueous Injections (hereinafter, simply referred to as an injection container grade)". In addition, the container 10 and the lid 30 may be formed of various other resin materials satisfying the same specification.

In particular, a material in which the shape is deformed in a case of the deformable portion 14 receiving an external force and the deformed shape is maintained is selected from among these materials. The material to be used is determined in consideration of the shape and the thickness of the deformable portion 14 and the magnitude of the external force required for the deformation.

Further, as illustrated in Fig. 2, it is preferable that the protrusions 12B are arranged closer to the side of the opening 12A than the side of the deformable portion 14. This means that, in a case where the distance from the opening 12A to the protrusion 12B and the distance from the position where the deformable portion 14 intersects with the accommodating portion 12 to the protrusion 12B are compared with each other, the distance from the opening 12A to the protrusion 12B is shorter than the other distance.

In a case where the protrusions 12B are provided on the side of the opening 12A, the needles 44 of the micro-needle array 40 are close to the skin 60. In a case where the micro-needle array 40 passes through the protrusions 12B and is pushed out from the accommodating portion 12, the skin 60 is immediately punctured by the needles 44, and thus the skin 60 can be stably punctured by the micro-needle array 40.

Fig. 11 is a cross-sectional view illustrating a micro-needle array unit 3 in still another form. The configurations which are the same as those of the micro-needle array unit 1 are denoted by the same reference numerals, and the description thereof will not be provided.

A difference between the micro-needle array unit 3 and the micro-needle array unit 1 is the shape of the deformable portion 14.

In the micro-needle array unit 3, the deformable portion 14 has a convex shape with the vertex portion 14A and has a dome shape. The dome shape indicates a shape having a curved surface with a certain curvature radius and examples thereof include a hemispherical shape. However, the example is not limited to the hemispherical shape and the curvature radii are not necessarily the same in the entirety of the shape.

The micro-needle array unit 3 which includes the deformable portion 14 having dome shape can exhibit the same effects as those of the micro-needle array unit 1.

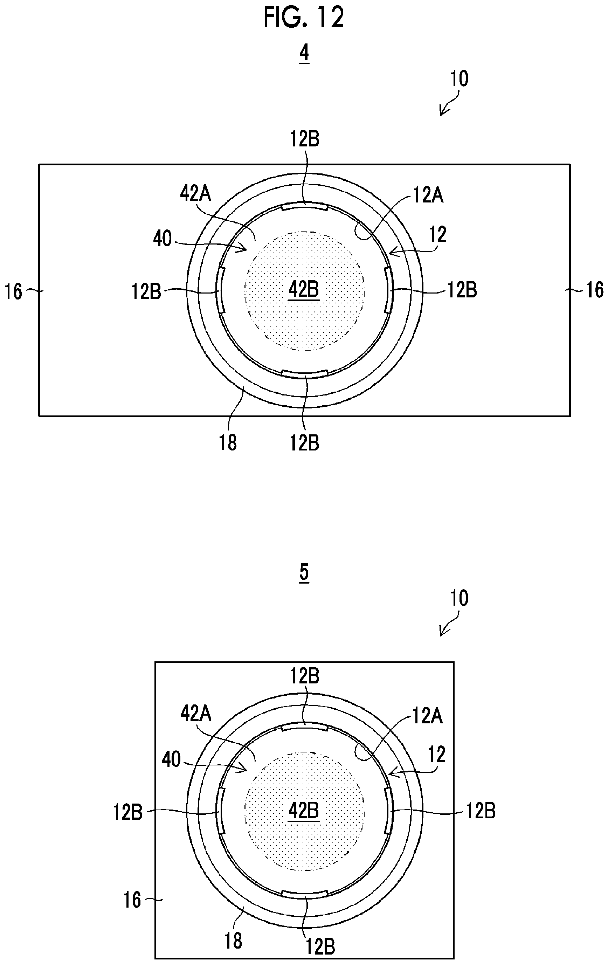

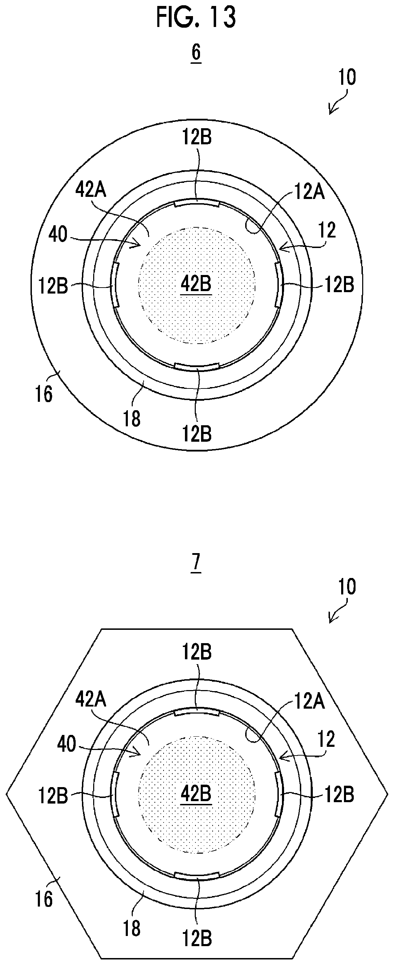

Fig. 12 show bottom views of the micro-needle array units 4 and 5 in other forms, and Fig. 13 show bottom views of the micro-needle array units 6 and 7 in other forms.

The configurations which are the same as those of the micro-needle array unit 1 are denoted by the same reference numerals, and the description thereof will not be provided.

As illustrated in Fig. 12, a difference between the micro-needle array unit 4 and the micro-needle array unit 1 is the shape of the flange portion 16. The micro-needle array unit 4 has a rectangular shape. Further, a difference between the micro-needle array unit 5 and the micro-needle array unit 1 is the shape of the flange portion 16. The micro-needle array unit 5 has a square shape.

As illustrated in Fig. 13, a difference between the micro-needle array unit 6 and the micro-needle array unit 1 is the shape of the flange portion 16. The micro-needle array unit 6 has a circular shape. Further, a difference between the micro-needle array unit 7 and the micro-needle array unit 1 is the shape of the flange portion 16. The micro-needle array unit 7 has a polygonal shape, which is a hexagon.

The micro-needle array units 4, 5, 6, and 7 having the flange portions 16 in shapes different from one another can exhibit the same effects as those of the micro-needle array unit 1. In Figs. 12 and 13, the lid 30 is not illustrated.

Basically, the flange portions 16 are attached to the skin. In a case where the shapes of the flange portions 16 are different from one another, this means that the areas where the flange portions 16 are in contact with the skin are different from one another.

It is preferable to select the container 10 that includes the flange portion 16 in an appropriate shape in consideration of the location where the skin is punctured by the micro-needle array 40 or the like.

Further, Fig. 12 and Fig. 13 illustrate a plurality of flange portions 16 having shapes different from one another, but the shapes are not limited thereto.

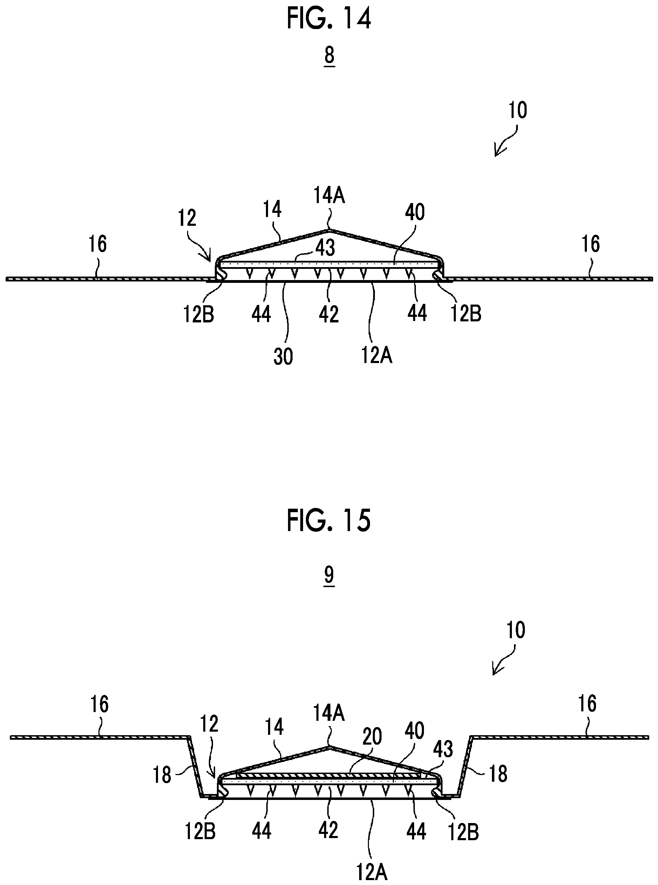

Fig. 14 is a cross-sectional view illustrating a micro-needle array unit 8 in still another form. As illustrated in Fig. 14, a difference between the micro-needle array unit 8 and the micro-needle array unit 1 is the shape of the flange portion 16. In the container 10 of the micro-needle array unit 8, the flange portion 16 does not include a bent portion. The flange portion 16 extends to the outside from the position of the opening 12A of the accommodating portion 12. The flange portion 16 is formed to be parallel to the sheet of the micro-needle array 40. The concept of parallel includes parallel and substantially parallel. The micro-needle array unit 8 is capable of further reducing the pressure between the micro-needle array 40 and the skin as compared to the micro-needle array unit having a bent portion.

The micro-needle array unit 8 having the flange portions 16 in a shape different from other shapes can exhibit the same effects as those of the micro-needle array unit 1.

Fig. 15 is a cross-sectional view illustrating a micro-needle array unit 9 in still another form. As illustrated in Fig. 15, the micro-needle array unit 9 is different from the micro-needle array unit 1 in terms that the micro-needle array unit 9 comprises a flat plate 20 on a side of the other surface 43 of the micro-needle array 40. The flat plate 20 and the container 10 may be separate members or the flat plate 20 may be integrated with the container 10.

The deformable portion 14 is deformed due to the external force and the deformed deformable portion 14 presses the micro-needle array 40 into the skin (not illustrated) through the flat plate 20. The entire surface of the micro-needle array 40 can be uniformly pressed by the flat plate 20. The micro-needle array unit 9 can exhibit the same effects as those of the micro-needle array unit 1.

The three-dimensional puncture instrument 70 having two vertically different areas, illustrated in Fig. 8, will be described. The three-dimensional shape of the puncture instrument 70 is not limited as long as the three-dimensional shape has two vertically different areas. It is preferable that the three-dimensional puncture instrument 70 having two vertically different areas is the puncture instrument 70 having protrusions on a flat plate or a frustum-like puncture instrument. In addition, the term "vertically" indicates that in a case where the largest area of the three-dimensional shape is placed on a lower side (downside in the gravity direction), the area positioned opposite to the largest area of the three-dimensional shape is placed on an upper side (upside in the gravity direction). Further, a three-dimensional shape having two vertically different areas is a three-dimensional shape in which the area of the uppermost surface thereof is different from the area of the lowermost surface thereof.

As illustrated in Fig. 8, in the three-dimensional puncture instrument 70 having two vertically different areas, the large area side between two vertically different areas is pressed by a finger or the like to allow the small area side to apply the external force in the direction of the opening. In this manner, the deformable portion is deformed, the other surface of the micro-needle array is pressed, the micro-needle array passes through the protrusions and is pushed out of the accommodating portion due to the pressing of the other surface, and the micro-needle array is pressed against the skin while the deformable portion maintains a deformed state. Here, since the micro-needle array is pressed against the skin by the small area side of the puncture instrument 70, the pressure increases, and the micro-needle array can sufficiently puncture the skin even in a case of being pressed by a finger. In this manner, the amount of the drug (the drug contained in the micro-needle array) that can be dissolved in the skin can be increased.

In a case where the large area side of the two vertically different areas of the puncture instrument 70 is pressed with a finger or the like, a pressure sensitive adhesive layer may be provided on the large area side in order to make the puncture instrument 70 adhere to the finger. As a pressure sensitive adhesive used for the pressure sensitive adhesive layer, a pressure sensitive adhesive containing an acrylic polymer or a rubber-based polymer can be used.

Examples of the acrylic polymer include polymers and copolymers containing at least one (meth)acrylic acid derivative typified by methyl acrylate, butyl acrylate, hydroxyethyl acrylate, 2-ethylhexyl acrylate, or 2-ethylhexyl methacrylate.

Examples of the rubber-based polymer include a styrene-isoprene-styrene block copolymer, isoprene rubber, polyisobutylene, a styrene-butadiene-styrene block copolymer, styrene-butadiene rubber, and polysiloxane.

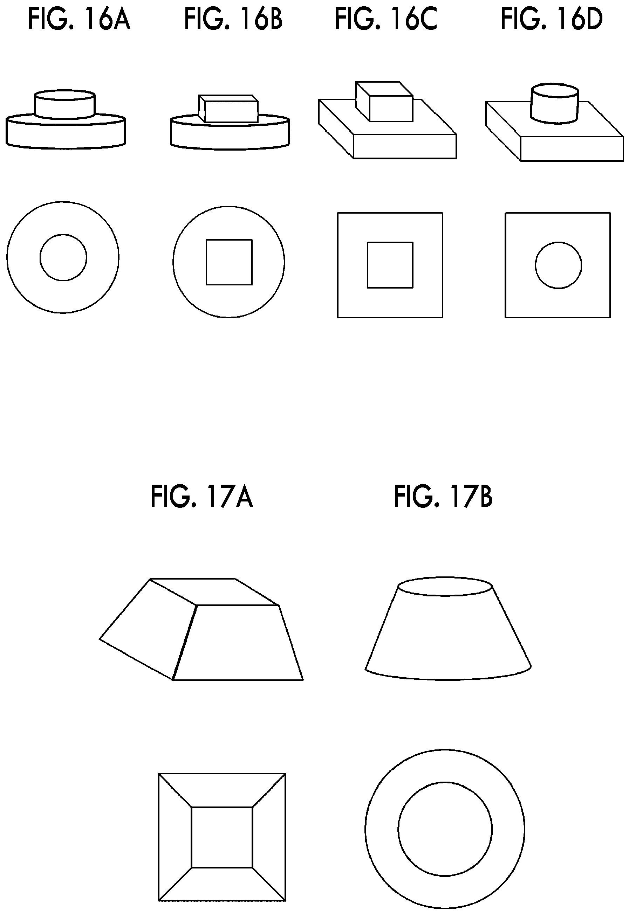

In the puncture instrument having a protrusion on a flat plate, the shape of the flat plate is not particularly limited, but the shape of the largest surface located in the vertical direction may be a circular shape, an elliptical shape, a triangular shape, a quadrangular shape, or a polygonal shape. Among these, a flat plate having a circular shape or a quadrangular shape is preferable. The shape of the protrusion on the flat plate is not particularly limited, but a flat plate having an area smaller than that of the flat plate may be used. Examples of the shape of the protrusion are the same as those for the shape of the flat plate described above. In Figs. 16A to 16D, as specific examples of the puncture instrument having a protrusion on a flat plate, examples in which the shape of the largest surface of the flat plate vertically located is circular or quadrangular and the shape of the largest surface of the protrusion is circular or quadrangular are described, but the present invention is not limited thereto.

In the frustum-like puncture instrument, the shape of the frustum is not particularly limited, and examples thereof include a truncated cone, a truncated pyramid, and a polygonal pyramid. Among these, a truncated cone or a square pyramid is preferable. Figs. 17A and 17B illustrate, as specific examples of the frustum-like puncture instrument, examples in which the shape of the frustum is a truncated cone and the shape thereof is a square pyramid are described, but the present invention is not limited thereto.

The size of the three-dimensional puncture instrument 70 having two vertically different areas is not particularly limited, but it is preferable that the diameter of one surface of the two vertically different areas is in a range of 3 mm to 10 mm and the diameter of the other surface of the two vertically different areas is in a range of 15 mm to 30 mm. Here, the diameter indicates the longest length passing through the center of the surface or the center of gravity. For example, in Fig. 16A, it is preferable that the diameter (corresponding to the diameter) of the circular flat plate located on the upper side is in a range of 3 mm to 10 mm and the diameter (corresponding to the diameter) of the circular flat plate located on the lower side is in a range of 15 mm to 30 mm.

The height (the size in the vertical direction) of the three-dimensional puncture instrument 70 having two vertically different areas is not particularly limited, but is preferably in a range of 5 mm to 50 mm, more preferably in a range of 5.5 mm to 40 mm, and still more preferably in a range of 6 mm to 30 mm. Here, the height (the size in the vertical direction) of the puncture instrument 70 indicates the length in the gravity direction passing through the centers of two vertically different surfaces or the centers of gravity. For example, in Fig. 16A, the length from the central surface of the circular flat plate located on the upper side to the central surface of the circular flat plate located on the lower side is preferably in a range of 5 mm to 50 mm.

The material of the three-dimensional puncture instrument 70 having two vertically different areas is not particularly limited, and a material having a hardness sufficient to push the micro-needle array out of the accommodating portion so that the micro-needle array is pressed against the skin is preferable. As the material having a hardness sufficient to press the micro-needle array against the skin, a material that is unlikely to be deformed by a load is preferable, a material having a tensile elastic modulus of 500 MPa or greater is more preferable, and a material having a tensile elastic modulus of 1000 MPa or greater is still more preferable. Specific examples of the material of the puncture instrument 70 include paper, paperboard, plastic, wood, glass, and metals. From the viewpoints of economy and ease of disposal, paper, paperboard, plastic, and wood are preferable, and paper, paperboard, plastic, and wood having a tensile elastic modulus of 500 MPa or greater or 1000 MPa or greater are more preferable as the material of the puncture instrument 70. The puncture instrument 70 can be produced by a known production technique such as compression molding, injection molding, forging, or casting depending on the material thereof.

In a case where paper or paperboard is selected as the material of the puncture instrument 70, hard paper or multi-layer paperboard obtained by coating one surface (or both surfaces) of hard paper with polyethylene or polypropylene can be used.

In a case where plastic is selected as the material of the puncture instrument 70, polyethylene, polypropylene, polystyrene, polycarbonate, acryl, polyethylene terephthalate (PET), and the like are preferable, and polypropylene, polystyrene, polycarbonate, acryl, and polyethylene terephthalate (PET) are more preferable. Hard paper or multi-layer paperboard obtained by coating one surface (or both surfaces) of hard paper with vinyl acetate, polyethylene, or polypropylene can be used.
1: micro-needle array unit
2: micro-needle array unit
3: micro-needle array unit
4: micro-needle array unit
5: micro-needle array unit
6: micro-needle array unit
7: micro-needle array unit
8: micro-needle array unit
9: micro-needle array unit
10: container
12: accommodating portion
12A: opening
12B: protrusion
14: deformable portion
14A: vertex portion
16: flange portion
18: bent portion
20: flat plate
30: lid
40: micro-needle array
41: sheet
42: one surface
42A: outer peripheral surface
42B: micro-needle region
42C: imaginary line
43: other surface
44: needle
50: finger
60: skin
70: puncture instrument

## Claims

1. A micro-needle array unit (1, 2, 3, 4, 5, 6, 7, 8, 9) comprising:
a micro-needle array (40) which has a sheet (41) and a plurality of needles (44) arranged inside an outer peripheral surface (42A) of one surface (42) of the sheet (41);
a container (10) which accommodates the micro-needle array (40) and includes an accommodating portion (12) having an opening (12A) and a protrusion (12B) that supports the outer peripheral surface (42A) of the micro-needle array (40), a deformable portion (14) disposed on a side opposite to the opening (12A) and integrated with the accommodating portion (12), and a flange portion (16) that is integrated with the accommodating portion (12) and brought into contact with a skin (60);
a lid (30) which seals the opening (12A) of the container (10); and
a three-dimensional puncture instrument (70) which has two vertically different areas,
**characterized in that**
wherein in a case where an external force applied in a direction of the opening (12A) by a smaller area between two vertically different areas of the puncture instrument (70) is received, the deformable portion (14) is deformed, and the other surface (43) of the micro-needle array (40) is pressed,
the micro-needle array (40) passes through the protrusion (12B) and is pushed out of the accommodating portion (12) due to the pressing of the other surface (43), and
the deformable portion (14) presses the micro-needle array (40) while maintaining a deformed state thereof.

2. The micro-needle array unit according to claim 1,
wherein the protrusion (12B) is disposed closer to a side of the opening than a side of the deformable portion.

3. The micro-needle array unit according to claim 1 or 2,
wherein the deformable portion (14) has a convex shape with a vertex portion separated from the micro-needle array (40).

4. The micro-needle array unit according to claim 3,
wherein the convex shape is a dome shape or a cone shape.

5. The micro-needle array unit according to any one of claims 1 to 4,
wherein a plurality of the protrusions are arranged at an equal interval in the accommodating portion.

6. The micro-needle array unit according to any one of claims 1 to 4,
wherein the protrusion (12B) is formed as a continuous protrusion disposed in the accommodating portion (12).

7. The micro-needle array unit according to any one of claims 1 to 5,
wherein the flange portion (16) has an adhesive on a side to be brought into contact with the skin (60).

8. The micro-needle array unit according to any one of claims 1 to 7, further comprising:
a flat plate (20) on a side of the other surface (43) of the micro-needle array.

9. The micro-needle array unit according to any one of claims 1 to 8,
wherein the flange portion (16) is provided in an entire circumference of the accommodating portion (12).

10. The micro-needle array unit according to any one of claims 1 to 9,
wherein the flange portion (16) includes a bent portion (18) that is bent to a side of the deformable portion (14).

11. The micro-needle array unit according to claim 10,
wherein the bent flange portion (18) is disposed at a position beyond the deformable portion (14) with reference to the opening (12A) of the accommodating portion (12).

12. The micro-needle array unit according to any one of claims 1 to 11,
wherein in the three-dimensional puncture instrument (70) having two vertically different areas, a diameter of a surface of one of the two vertically different areas is in a range of 3 mm to 10 mm, and a diameter of a surface of the other of the two vertically different areas is in a range of 15 mm to 30 mm.

## Patentansprüche

1. Mikronadel-Arrayeinheit (1, 2, 3, 4, 5, 6, 7, 8, 9), umfassend:
ein Mikronadelarray (40), das ein Flachstück (41) und eine Mehrzahl von Nadeln (44), die innerhalb einer Außenumfangsfläche (42A) einer Oberfläche (42) des Flachstücks (41) angeordnet sind, aufweist;
einen Behälter (10), der das Mikronadelarray (40) aufnimmt und einen Aufnahmeabschnitt (12) mit einer Öffnung (12A) und einem die Außenumfangsfläche (42A) des Mikronadelarrays (40) lagernden Vorsprung (12B), einen verformbaren Abschnitt (14), der auf einer Seite abgewandt von der Öffnung (12A) angeordnet und mit dem Aufnahmeabschnitt (12) integriert ist, und einen Flanschabschnitt (16), der mit dem Aufnahmeabschnitt (12) integriert und in Berührung mit einer Haut (60) zu bringen ist, enthält;
einen Deckel (30), der die Öffnung (12A) des Behälters (10) abdichtet; und
ein dreidimensionales Punkturinstrument (70), welches zwei vertikal unterschiedliche Bereiche aufweist,
**dadurch gekennzeichnet, dass** in dem Fall, dass eine in Richtung der Öffnung (12A) durch eine kleinere Fläche zwischen zwei vertikal unterschiedlichen Flächen des Punkturinstruments (70) aufgebrachte externe Kraft empfangen wird, der verformbare Abschnitt (14) verformt wird, und die andere Oberfläche (43) des Mikronadelarrays (40) gedrückt wird,
das Mikronadelarray (40) durch den Vorsprung (12B) hindurchtritt und aus dem Aufnahmeabschnitt (12) durch das Drücken der anderen Oberfläche (43) nach außen gedrückt wird, und
der verformbare Abschnitt (14) das Mikronadelarray (4) drückt, während sein verformter Zustand aufrecht erhalten wird.

2. Mikronadel-Arrayeinheit nach Anspruch 1,
bei der der Vorsprung (12B) näher an einer Seite der Öffnung angeordnet ist als an einer Seite des verformbaren Abschnitts.

3. Mikronadel-Arrayeinheit nach Anspruch 1 oder 2,
bei der der verformbare Abschnitt (14) eine konvexe Form mit einem von dem Mikronadelarray (4) abgerückten Scheitelabschnitt aufweist.

4. Mikronadel-Arrayeinheit nach Anspruch 3,
bei der die konvexe Form eine Dom-Form oder eine konische Form ist.

5. Mikronadel-Arrayeinheit nach einem der Ansprüche 1 bis 4,
bei der eine Mehrzahl von Vorsprüngen in gleichen Intervallen innerhalb des Aufnahmeabschnitts angeordnet ist.

6. Mikronadel-Arrayeinheit nach einem der Ansprüche 1 bis 4,
bei der der Vorsprung (12B) als kontinuierlicher Vorsprung ausgebildet ist, angeordnet in dem Aufnahmeabschnitt (12).

7. Mikronadel-Arrayeinheit nach einem der Ansprüche 1 bis 5,
bei der der Flanschabschnitt (16) auf einer in Berührung mit der Haut (6) zu bringenden Seite einen Klebstoff aufweist.

8. Mikronadel-Arrayeinheit nach einem der Ansprüche 1 bis 7, weiterhin umfassend:
eine flache Platte (20) auf einer Seite der anderen Oberfläche (43) des Mikronadelarrays.

9. Mikronadel-Arrayeinheit nach einem der Ansprüche 1 bis 8,
bei der der Flanschabschnitt (16) in einem Gesamtumfang des Aufnahmeabschnitts (12) vorhanden ist.

10. Mikronadel-Arrayeinheit nach einem der Ansprüche 1 bis 9,
bei der der Flanschabschnitt (16) einen gebogenen Abschnitt (18) enthält, der zu einer Seite des verformbaren Abschnitts (14) hin gebogen ist.

11. Mikronadel-Arrayeinheit nach Anspruch 10,
bei der der gebogene Flanschabschnitt (18) an einer Stelle jenseits des verformbaren Abschnitts (14) bezüglich der Öffnung (12A) des Aufnahmeabschnitts (12) angeordnet ist.

12. Mikronadel-Arrayeinheit nach einem der Ansprüche 1 bis 11,
bei der in dem dreidimensionalen Punkturinstrument (70) mit zwei vertikal unterschiedlichen Bereichen ein Durchmesser einer Oberfläche von einem der zwei vertikal unterschiedlichen Bereiche in einem Bereich von 3 mm bis 10 mm liegt, und ein Durchmesser einer Oberfläche des anderen der beiden vertikal unterschiedlichen Bereiche in einem Bereich von 15 mm bis 30 mm liegt.

## Revendications

1. Unité de réseau de micro-aiguilles (1, 2, 3 4, 5, 6, 7, 8, 9), comprenant :
un réseau de micro-aiguilles (40), lequel présente une feuille (41) et une pluralité d'aiguilles (44) agencées dans une surface périphérique extérieure (42A) d'une surface (42) de la feuille (41) ;
un récipient (10), lequel loge le réseau de micro-aiguilles (40) et inclut une portion de logement (12) présentant une ouverture (12A) et une saillie (12B), laquelle supporte la surface périphérique extérieure (42A) du réseau de micro-aiguilles (40), une portion déformable (14) disposée sur un côté face à l'ouverture (12A) et d'un seul tenant avec la portion de logement (12), et une portion de bride (16), laquelle est d'un seul tenant avec la portion de logement (12) et amenée en contact avec une peau (60) ;
un couvercle (30), lequel ferme l'ouverture (12A) du récipient (10), et
un instrument de ponction tridimensionnel (70), lequel présente deux zones différentes verticalement,
**caractérisée en ce que**
dans un cas où une force externe appliquée dans une direction de l'ouverture (12A) sur une zone plus petite entre deux zones différentes verticalement de l'instrument de ponction (70) est reçue, la portion déformable (14) est déformée, et l'autre surface (43) du réseau de micro-aiguilles (40) est pressée ;
le réseau de micro-aiguilles (40) passe à travers la saillie (12B) et est poussé hors de la portion de logement (12) en raison de la pression de l'autre surface (43), et
la portion déformable (14) presse le réseau de micro-aiguilles (40), tout en maintenant un état déformé de celui-ci.

2. Unité de réseau de micro-aiguilles selon la revendication 1,
dans laquelle la saillie (12B) est disposée plus près d'un côté de l'ouverture qu'un côté de la portion déformable.

3. Unité de réseau de micro-aiguilles selon la revendication 1 ou 2,
dans laquelle la portion déformable (14) présente une forme convexe avec une portion de sommet séparée du réseau de micro-aiguilles (40).

4. Unité de réseau de micro-aiguilles selon la revendication 3,
dans laquelle la forme convexe est une forme de dôme ou une forme de cône.

5. Unité de réseau de micro-aiguilles selon l'une quelconque des revendications 1 à 4,
dans laquelle une pluralité des saillies sont agencées à intervalle égal dans la portion de logement.

6. Unité de réseau de micro-aiguilles selon l'une quelconque des revendications 1 à 4,
dans laquelle la saillie (12B) se présente sous la forme d'une saillie continue disposée dans la portion de logement (12).

7. Unité de réseau de micro-aiguilles selon l'une quelconque des revendications 1 à 5,
dans laquelle la portion de bride (16) présente un adhésif sur un côté à amener en contact avec la peau (60).

8. Unité de réseau de micro-aiguilles selon l'une quelconque des revendications 1 à 7, comprenant en outre :
une plaque plate (20) sur un côté de l'autre surface (43) du réseau de micro-aiguilles.

9. Unité de réseau de micro-aiguilles selon l'une quelconque des revendications 1 à 8,
dans laquelle la portion de bride (16) est prévue sur toute la circonférence de la portion de logement (12).

10. Unité de réseau de micro-aiguilles selon l'une quelconque des revendications 1 à 9,
dans laquelle la portion de bride (16) inclut une portion courbée (18), laquelle est courbée vers un côté de la portion déformable (14).

11. Unité de réseau de micro-aiguilles selon la revendication 10,
dans laquelle la portion de bride courbée (18) est disposée sur une position au-delà de la portion déformable (14) avec référence à l'ouverture (12A) de la portion de logement (12).

12. Unité de réseau de micro-aiguilles selon l'une quelconque des revendications 1 à 11,
dans laquelle dans l'instrument de ponction tridimensionnel (70) présentant deux zones différentes verticalement, un diamètre d'une surface d'une des deux zones différentes verticalement est compris dans une plage allant de 3 mm à 10 mm, et un diamètre d'une surface de l'autre surface des deux zones différentes verticalement est compris dans une plage allant de 15 mm à 30 mm.
